# EUROPEAN PATENT APPLICATION

(11) **EP 2 444 022 A2**
(43) Date of publication of application: **25.04.2012**
(21) Application number: 11185488.1
(22) Date of filing: 17.10.2011
(51) Int. Cl.: A61B 19/02

(54) **Medical bag**

(30) Priority: 20.10.2010 GB 1017650; 21.03.2011 GB 1104749
(71) Applicant: The Royal College of Art, London SW7 2EU (GB); Swann, David, Huggate YO42 1YE (GB)
(72) Inventor: Swann, David, Huggate, YO42 1YE (GB)
(74) Representative: Hedley, Nicholas James Matthew

(57) **Abstract**

A medical bag is described that has a carrying configuration (Figure 5) and a dispensing configuration (Figure 1) and comprises:
- a handle (20) for carrying the bag,
- a space (12) within the bag for accommodating a tapered storage drawer (14), the space tapering from a wide end(16) to a narrow end (18) and the drawer being of a corresponding shape to the space,

When the bag is carried by the handle (20) in the carrying configuration (Figure 5), the bottom surface (17) of the space slopes down towards the narrow end so that gravity acts to force the drawer towards the narrow end of the space; however, because of its size, the drawer (14) cannot pass through the narrow end of the space, thereby retaining the tapered storage drawer within the space.

## Description

### Technical Field

This invention relates to a product that stores and transports items necessary for providing medical care, including emergency care, urgent care, planned care, maternity nursing, sample taking, health demonstrations, veterinary care and other functions performed by healthcare professionals.

### Background Art

The nursing bag is an essential tool for healthcare professionals when delivering care to patients or animals outside of hospitals and in the community or patient homes.

In hospitals, the medical trolley is an essential item of equipment to support the delivery of treatments by providing a sterile lay-down surface for clinical items. Unlike hospitals every patient's home is different and therefore seen as an uncontrollable clinical environment. Clinicians delivering treatments in this context are required to improvise as they often work off the floor, from a table or off a chair seat. Conventional nursing bags do not provide a professional, safe and consistent work zone to support the preparation and delivery of clinical treatments. Furthermore research evidence highlights: (i) patient safety concerns as traditional nursing bags are manufactured from permeable materials that harbour bacteria; (ii) bags utilising traditional zips, clips and fasteners inhibits effective hand-cleaning to minimise the presence of bacteria; (iii) separation of treatment space from storage receptacles exacerbates the problem of excessive bending and reaching during treatment delivery; (iv) storage of items in pockets inhibits the quick identification and retrieval of items; leading to nurses searching through pockets to find required items.

The Health & Safety at Work Act (1974) provides a statutory legal requirement for employers to protect the health, safety and welfare of employees at work. The wellbeing of employees is enhanced further through the Provision and Use of Work Equipment (1998); a statutory law requiring employers to ensure that workers are equipped with safe and fit for purpose equipment to perform their roles.

Patient safety is a prerequisite quality of any medical device. Hospital acquired infections (HAls) such as Meticillin-Resistant Staphylococcus Aureus (MRSA) are now considered to be a main disease threat in Europe. Research suggests the proliferation of drug-resistance infections in the community such as MRSA makes nursing bags both outdated and dangerous. The National Patient Safety Agency does not specify direct guidelines for the cleaning of nursing bags used in the community. The absence of a structured cleaning regime together with the high number of patients nurses visited each day (<17) raised concerns for the transfer/ harbouring of pathogens.

Observations revealed the absence of equipment specifically designed to support professionals working in this inconsistent setting. Fieldwork activities captured the practice of using bags specifically designed for non-healthcare applications: camera bags, plastic toolboxes and accountant cases.

### Disclosure of the Invention

According to the present invention, there is provided a medical bag that has a carrying configuration and a dispensing configuration and comprises:
- a handle for carrying the bag,
- a space within the bag for accommodating a tapered storage insert, the space tapering from a wide end to a narrow end,
wherein, when the bag is carried by the handle in the carrying configuration, the space has a bottom surface for supporting the insert and wherein the bottom surface slopes downwardly towards the narrow end of the tapered space so that gravity acts to force the insert towards the narrow end of the space, thereby retaining the tapered storage insert within the space.

As used herein, the term "configuration" includes the orientation of the medical bag as well as the situation in which the overall shape of the bag is changed from one configuration to another.

The essence of the present invention lies in the provision of a tapered insert within a medical bag and the provision of a correspondingly shaped space for accommodating the tapered insert. When the medical bag is carried by the handle, the insert should be on its side supported by the sloping bottom surface of the space, which means that the insert is urged by gravity towards the narrow end of the tapered space, thereby holding the drawing in place without the use of fasteners.

By avoiding the use of fasteners, a very simple case carcass can be provided that can readily be cleaned and disinfected. To that end, the narrow end and the wide end of the space are preferably open so that there are no corners in which infectious materials can be located and which are difficult to clean out. Obviously, the insert is inserted into the space through the wide end of the space and the narrow end of the space is too small to allow the insert to pass out of the space and indeed it is preferred that the narrow end on the space should be the same size or smaller than the small end of the tapering insert.

The bottom surface of the space could slope towards the narrow end of the space by constructing the bag with such a sloping surface both when the bag is being carried by the handle and when it is resting on a horizontal surface. Alternatively, the surface could slope only when the bag is lifted by the handle, which may be achieved by locating the handle on one side of the centre of gravity of the bag so that the bag is tipped when being carried by the handle and, in this tipped orientation, the bottom surface of the space will be correspondingly tipped to slope towards the narrow end of the tapered space so that gravity acts to force the insert towards the narrow end of the space to retain the insert within the space. In this latter arrangement, the bottom surface of the space can be parallel to the base of the case when the case is resting on a horizontal surface. It will be appreciated that both the above arrangements can be provided in a single bag.

The bag preferably has a rigid carcass in which the tapering space is formed. The carcass may be in the form of a one-piece plastic moulding.

The term "insert" used herein includes a drawer that a practitioner fills with contents that he wants, and also includes a pre-filled drawer that is provided by a supplier pre-filled with contents. It also includes a pack (i.e. without a surrounding drawer or container) that can be inserted into and withdrawn from the space(s) in the bag; an example of such an insert pack is a wound dressing pack that is held by a wrapping to form a pack that has a shape corresponding to the shape of the space. Thus an "insert" is anything that can be inserted into the space of the bag and withdrawn from it. Although the insert (including a drawer or pack mentioned above) preferably has a tapered shape, this is not necessary if it is small enough to be inserted into the wide end of the space and is large enough that it cannot pass out of the narrow end of the space.

As well as providing materials, pharmaceuticals, equipment etc that can be used by the practitioner during a visit to a patient, the bag can also be used to deliver materials, pharmaceuticals, equipment etc to the patient for use after the visit is over and also to remove hazardous material or equipment for safe disposal later, e.g. a biological hazard or a "sharp" such as a catheter or syringe needle, which avoids the patient disposing of such hazards with the household waste.

In one embodiment, the medical bag has two halves connected by a hinge. Each half may have a surface immediately adjacent to the hinge and the bag can be opened up into a dispensing configuration in which the two surfaces lie side-by-side to form a horizontal treatment surface. However, the bag can be closed or folded up into the carrying configuration in which the two surfaces of the halves are vertical and lie flat against each other.

The two halves of the bag can be constructed from a single plastic moulding and the hinge can be a living hinge.

The handle can be in the form of an aperture in the two halves so that, when the bag is folded up into its carrying configuration, the handle apertures lie in register, thus forming a single handle.

In the dispensing configuration, the inserts can lie underneath the treatment surface and provide a support for it, thereby elevating it above ground-level. In the dispensing configuration, the inserts can readily be removed from the spaces within the bag to gain access to medical equipment stored therein.

An important aspect of the present invention is that it can be made without internal or external protrusions and without zips, clips and fasteners that can inhibit effective cleaning of the medical case.

### Brief Description of the Drawings

There will now be described, by way of example only, two embodiments of the present invention by reference to the accompanying drawings in which:
Figure 1 shows the bag in a dispensing configuration;
Figure 2 shows the bag in a carrying configuration;
Figure 3 shows the bag in the carrying configuration but resting in the ground;
Figure 4 shows the configuration of the bag when lifted by the handle;
Figure 5 is the same as Figure 4 but shows, in cross hatching, the position of the drawer within the bag; Figure 6 shows the bag without the drawers;
Figure 7 shows a second embodiment ot the bag in a dispensing configuration;
Figures 8 and 9 show a plan view and an end view of the bag of Figure 7 in a dispensing configuration;
Figures 10 and 11 show a perspective view and an end view of the bag of Figure 7 in the folded up or carrying configuration when standing on the ground; and
Figures 12 to 14 show a side view, a plan view and a perspective view of a drawer for use with the bag.

### Detailed Description of the Invention

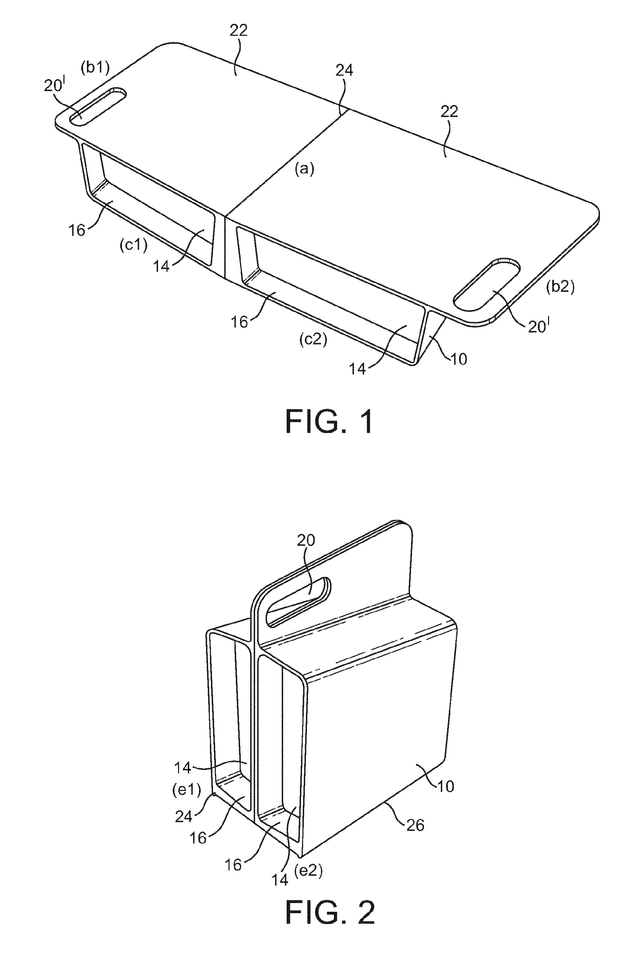

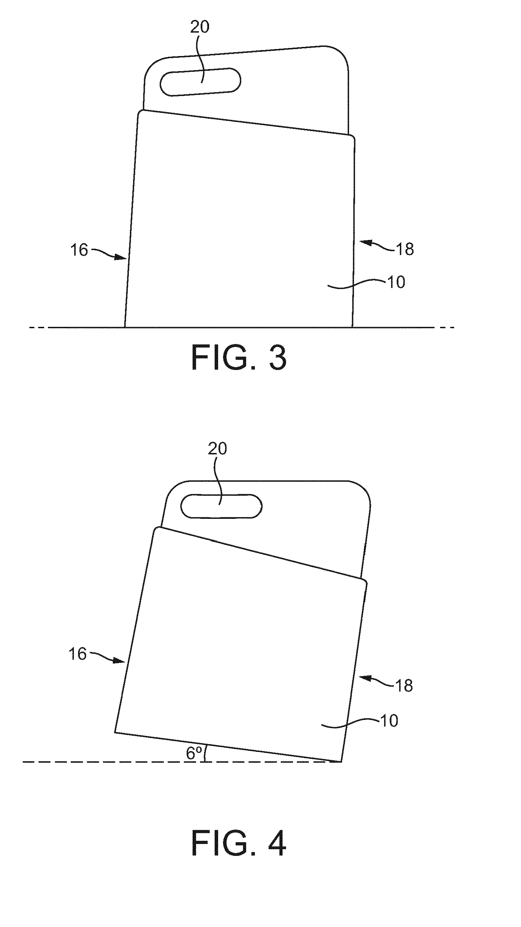

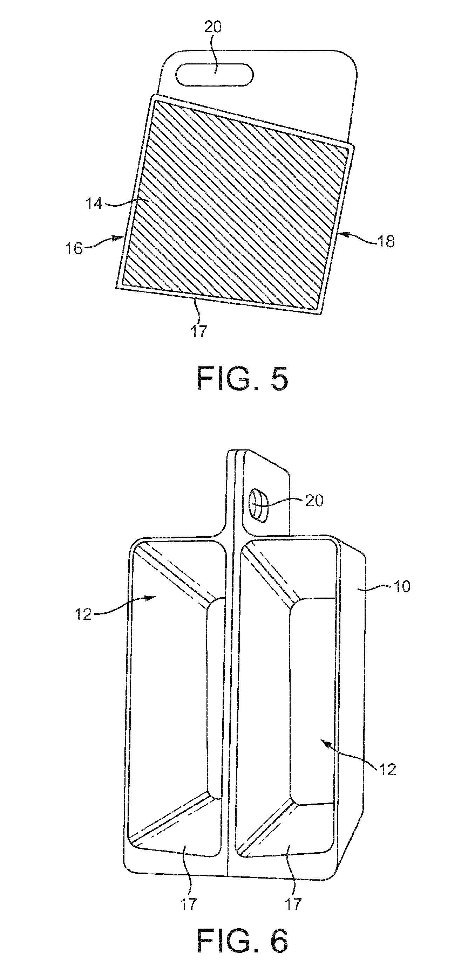

Figures 2 to 6 show the bag in a carrying or folded-up configuration. As can be seen, the bag has a rigid carcass (see Figure 6) having two side-by-side spaces 12 for accommodating drawers 14 (see Figure 2). The drawers can contain medical supplies and can be withdrawn from the spaces in order to gain access to the contents of the drawers.

The spaces 12 taper from the front of the case 16 to the back of the case, that is to say the height of the space is greater at the front end 16 than at the back end 18. The drawers 14 have a correspondingly tapered shape so that they can fit in the space and be withdrawn through the front of the case 16 but not through the back of the case 18. This configuration can be seen especially well in Figure 5 where the drawers 14 is shown in crossed hatching.

When resting on the ground, the bottom surface 17 of the spaces 12 may also be horizontal (as shown) or can slope towards the narrow end of the space (not shown). In the former case, the bag must be tilted when it is picked up and this can be brought about by providing a handle 20 for carrying the case that is arranged to one side of the centre of gravity of the case so that it tips when picked up by the handle. The tipping angle should be sufficient to ensure that the drawers 14 do not fall out of the space 12 when being carried and we have found that an angle of 4 to 10 degrees, e.g. 6 degrees, is sufficient for this purpose.

The arrangement of providing a sloping bottom surface when the case is being carried by the handle allows the bag to dispense with fasteners to retain the drawers within the spaces; such fasteners can be difficult to clean and can harbour microorganisms.

The case is made in two halves so that it can be opened up to a dispensing configuration shown in Figure 1. As can be seen, each half has a surface 22 that lie side-by-side and that can be used by the medical practitioner to support medical equipment, provide a surface for dressing etc. In this connection, it should be noted that the two surfaces 20 are brought face-to-face when the bag is folded into the carrying configuration shown in Figures 2 to 6 and therefore the support surfaces 22 are protected from infections, thereby helping to maintain the cleanliness and sterility of the surfaces.

As is also clear from Figure 1, the surfaces 20 extend beyond the drawers to provide a long treatment surface. In these extensions, handle openings 20' are provided that, when the case is folded up into the carrying configuration (Figures 2 to 6), the two openings 20' are brought into register to form a single carrying handle 20.

In order to maintain the sterility of the combined surface 22, the two surfaces may be joined by a living hinge 24 which provides a continuity in the surface 22 from one half of the case to the other and therefore can be kept clean by wiping and disinfection. Obviously, the case can hinge about the living hinge 24 when transformed from the dispensing configuration of Figure 1 to the carrying configuration of Figures 2 to 6. The whole of the carcass 10 can be formed in a single moulded article, which includes the living hinge 24.

As can be seen especially in Figure 6, the spaces 12 are open both at the front and at the back so that no corner is provided that can harbour infectious microorganisms. Furthermore, as discussed above, no fasteners are required in order to keep the drawers 14 in the spaces 12 when the case is being carried, thereby further increasing the ease of cleaning.

Ridges 26 are provided along the outside edges of the base of the case, thereby elevating the outside edges above the level of the centre of the base of the case, which keeps the case in its closed configuration shown in Figures 2 to 6 when it stands on a surface. Obviously, instead of ridges 26, it is possible to provide individual projections or feet along the outer edge of the base of the case that perform the same function.

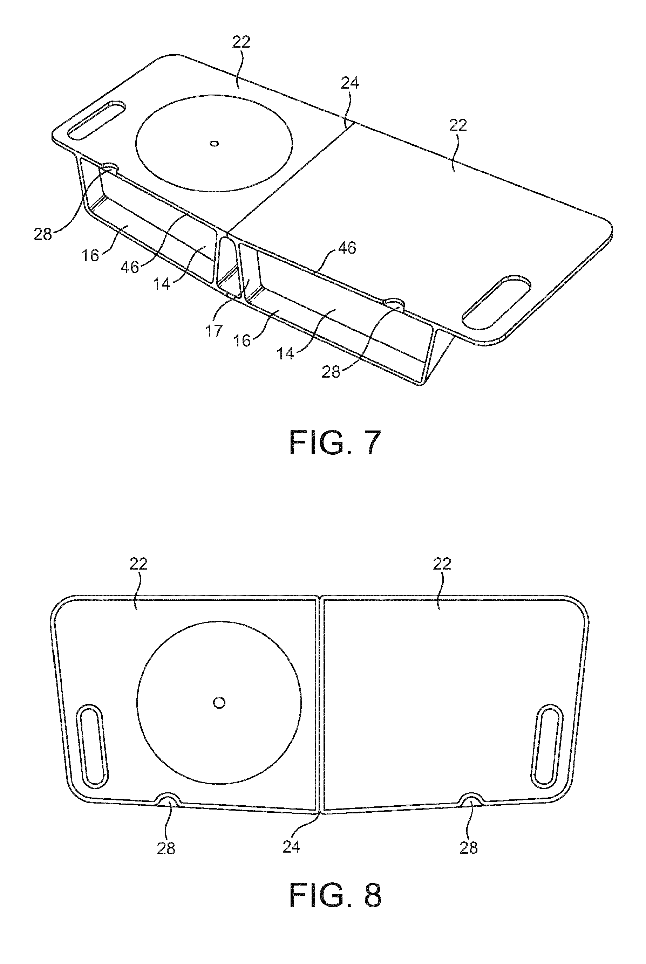

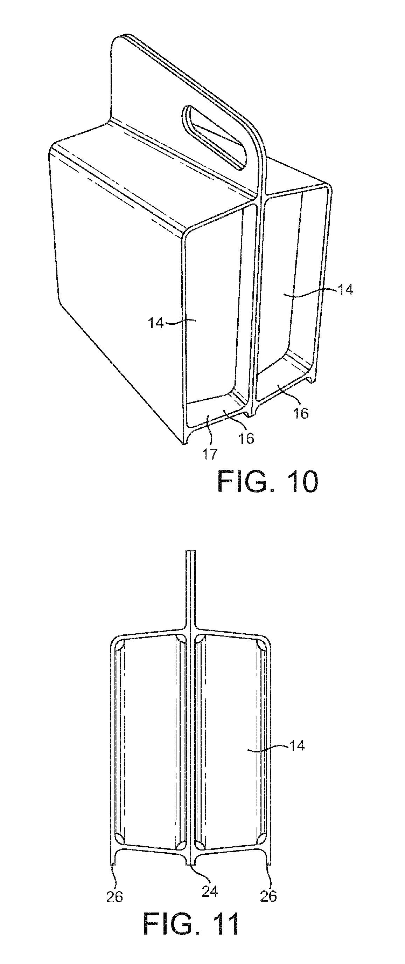

A slightly different second embodiment is shown in Figures 7 to 11, where the same reference numbers are used to refer to corresponding parts. The second embodiment differs from the first embodiment in having notches 28 to assist in removing drawers 14 from the spaces 12.

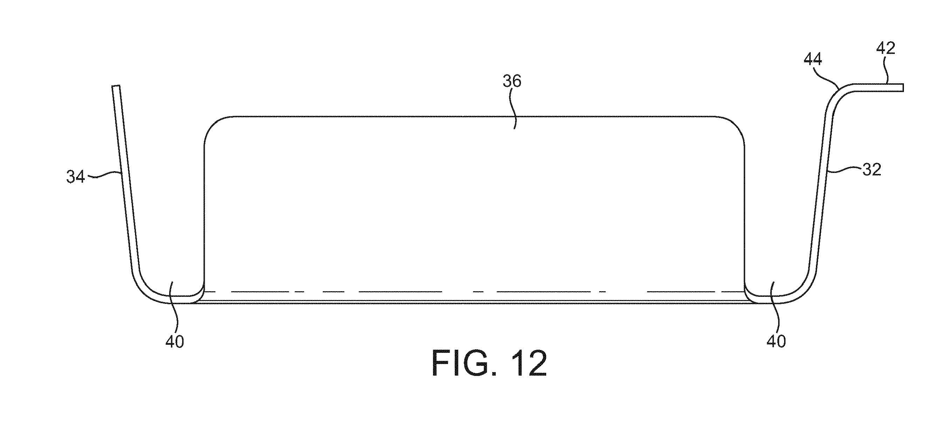

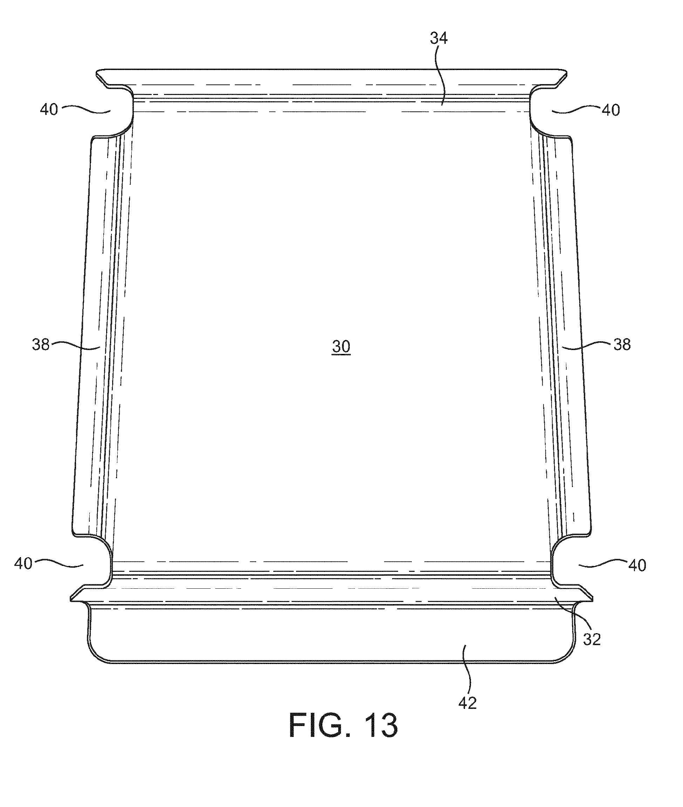

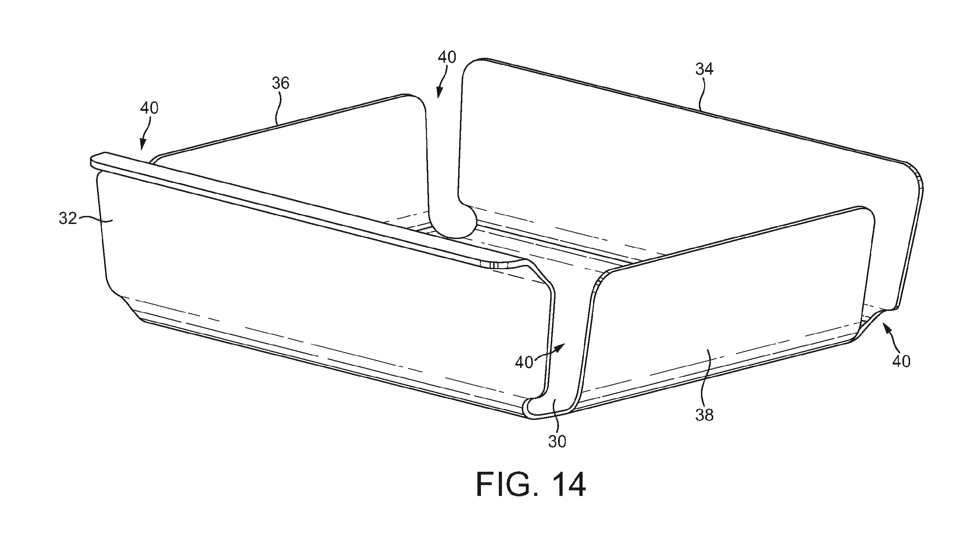

The drawers are shown in Figures 12 to 14 and have a base 30, a front wall 32, a back wall 34 and sidewalls 36,38 that surround the base on all four sides. However, the walls are not connected and there are gaps 40 between them to avoid providing hard-to-clean corners. As can be seen from Figure 13, the drawer tapers from the front end wall 32 to the back end wall 34 so that it has a corresponding shape to the tapered spaces 12 in the bag. A lip 42 extends horizontally from the top of the front wall 32 and is Joined to the wall 32 by a curved section 44.

Returning to Figures 7 to 11, the drawers 14 slide into the spaces 12 back end 34 first; when fully inserted the lip 40 can be grasped in the region of the notches 28 to remove the drawers. It is possible that the lip is marginally taller than the top 46 of the space 12, as seen in Figure 7, so that it frictionally engages the top 46 when the drawer 14 is slid into the space 12 and so helps retain the drawer in the space. The drawer can be smoothly inserted into the gap 12 of the bag because the front wall 32 of the drawer is flexible and the curved section 42 engages the top 46 of the space and so depresses the lip 42 slightly to allow the lip to be moved readily under the top 46.

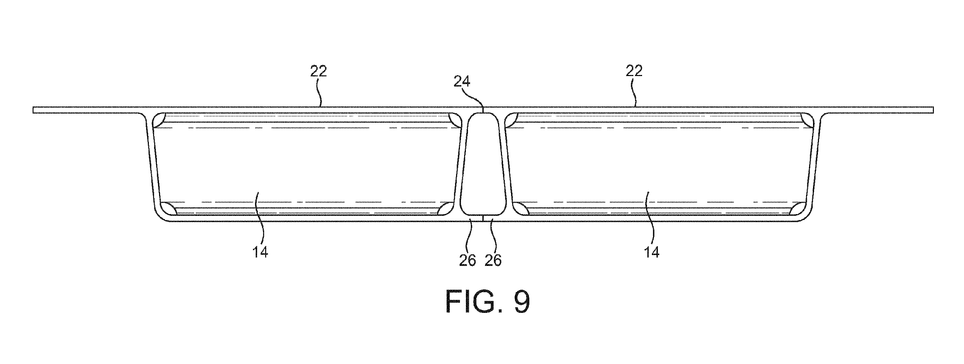

In addition, the ridges 26 in the second embodiment so that, in addition to keeping the bag closed when standing on a surface, the two opposing ridges engage with each other when in the dispensing configuration (see Figure 9) to provide a rigid structure in the dispensing configuration and to prevent the hinge 24 being flexed beyond 180°.

It should be noted that the drawer 14 (including lip 40) is a single part moulding. The walls 32,34,36,38 do not join up and so there are no edges or corners between them. It also uses curved connections between the base 30 and the walls and between the lip 40 and the front wall 32. Thus the drawer avoids the use of angular/sharp edges and corners that are difficult to reach and clean thoroughly and that could act as a bacteria/dirt trap. The avoidance of angular/sharp edges and corners is also true of the bag as a whole.

The drawers can be filled by a practitioner with any contents that he wants; alternatively, drawers could be provided by a supplier that are pre-filled with contents.

Although the above description in connection with the Figures has described the use of drawers, it will be appreciated from the earlier, more general description that inserts other than drawers can be used instead. Thus, the insert may be a pack (i.e. without a surrounding drawer or container) that can be inserted into and withdrawn from the space(s) 12 in the bag, for example the insert may be wound dressings that are held by a wrapping to form a pack that has a shape corresponding to the shape of the space.

## Claims

1. A medical bag that has a carrying configuration and a dispensing configuration and comprises:
- a handle (20) for carrying the bag,
- a space (12) within the bag for accommodating a tapered storage insert, the space tapering from a wide end (16) to a narrow end (18),
wherein, when the bag is carried by the handle in the carrying configuration, the space (12) has a bottom surface (17) for supporting the insert and wherein the bottom surface slopes downwardly towards the narrow end (18) of the tapered space so that gravity acts to urge the insert towards the narrow end (18) of the space, thereby retaining the insert within the space.

2. A medical bag as claimed in claim 1, which includes said tapered storage insert (14), the storage insert having a shape corresponding to the tapered shape of the space (12) and tapering from a wide insert end to a narrow insert end, the width of the wide insert end being larger than the space narrow end (18) so that the insert cannot pass through the space narrow end.

3. A medical bag as claimed in claim 1 or claim 2, wherein the insert (14) is a drawer the drawer (14) optionally having a base (30) and individual side walls (32, 34. 36, 38) arranged around the base and wherein the walls are separated from each other by gaps (40) at the corners of the drawer.

4. A medical bag as claimed in claim 3 or claim 4, wherein the drawer (14) has a part (40) that engages a wall defining the space (12) when the drawer is inserted into the space, thereby assisting in retaining the drawer in the space.

5. A medical bag as claimed in any preceding claim, having a front (16) and a back (18) and wherein the space tapers from the front to the back the space (12) optionally being is open at its front (16) and back (18).

6. A medical bag as claimed in any preceding claim, wherein the space (12) has a truncated wedge-shape, one dimension of which is smaller than its other two dimensions and wherein said smaller dimension forms the height of the space when the bag is in the dispensing configuration and forms the width of the space when the bag is in the carrying configuration.

7. A medical bag as claimed in any preceding claim, wherein the position of the handle (20) is such that, when the bag is carried by the handle in the carrying configuration, the bag tilts in such a way that the bottom surface (17) of the space slopes towards the narrow end of the space so that gravity acts to urge the insert into the space towards the narrow end, thereby retaining the tapered storage insert within the space.

8. A medical bag as claimed in any preceding claim, wherein, when the bag is carried by the handle in the carrying configuration, the bag tilts such that the base is orientated at an angle of 4 to 10°, e.g. 6° relative to the horizontal.

9. A medical bag as claimed in any preceding claim, which includes a base on which the bag can stand when in the carrying configuration and wherein said lower space surface (17) is parallel to the base or slopes relative to the base towards the narrow end of the space.

10. A medical bag as claimed in any preceding claim which has a carcass for accommodating the insert and wherein the carcass is rigid.

11. A medical bag as claimed in any preceding claim, which has no fasteners for holding the storage insert.

12. A medical bag as claimed in any preceding claim, which has two halves connected by a hinge (24), each half having a surface (22) immediately adjacent to the hinge and wherein the bag can be pivoted about the hinge from the carrying configuration to the dispensing configuration and, when in the dispensing configuration, the two surfaces (22) lie side-by-side to form a single treatment surface and, when in the carrying configuration, the two surfaces (22) lie flat against each other
wherein said bag is optionally constructed from one plastic moulding incorporating a living hinge (24).

13. A medical bag as claimed in claim 12, wherein each of the two halves includes a handle aperture (20') and when the bag is in the carrying configuration with the two halves being brought together, the two handle apertures lie in register thus forming one unified handle (20).

14. A medical bag as claimed in claim 12 or claim 13, which, when the bag is in the carrying configuration, it includes a base having edge protrusions (24) such that, when the bag is standing on a horizontal surface, the protrusions act to push the bag closed.

15. A medical bag as claimed in any one of claims claim 12 to 14, wherein the two halves each has a tapering space for accommodating a tapered storage insert.
